# EUROPEAN PATENT APPLICATION

(11) **EP 4 502 249 A1**
(43) Date of publication of application: **05.02.2025**
(21) Application number: 23780838.1
(22) Date of filing: 30.03.2023
(51) Int. Cl.: C25F 3/16, A61B 17/06, C22C 27/04, C25F 3/26, G01R 1/067

(54) **RHENIUM TUNGSTEN ALLOY WIRE, PRODUCING METHOD FOR SAME, MEDICAL NEEDLE, AND PROBE PIN**

(30) Priority: 30.03.2022 JP 2022055238
(71) Applicant: Kabushiki Kaisha Toshiba, Tokyo 105-0023 (JP); Toshiba Materials Co., Ltd., Isogo-Ku Yokohama-Shi Kanagawa 235-0032 (JP)
(72) Inventor: BABA, Hideaki, Yokohama-shi, Kanagawa 235-0032 (JP); AOYAMA, Hitoshi, Yokohama-shi, Kanagawa 235-0032 (JP); FUKUSHI, Daisuke, Yokohama-shi, Kanagawa 235-0032 (JP); FUJISAWA, Sachiko, Yokohama-shi, Kanagawa 235-0032 (JP)
(74) Representative: AWA Sweden AB
(86) International application number: PCT/JP2023/013101
(87) International publication number: WO 2023/190830

(57) **Abstract**

According to one embodiment, a rhenium-tungsten alloy wire is a wire made of a tungsten alloy containing rhenium. The amount of NH₄ at a wire surface is 10 massppm or less, and is according to a ratio of a weight of NH₄ ions at a wire surface to a weight of an alloy wire (a weight of NH₄ ions at a wire surface/a weight of an alloy wire). According to the embodiment, a medical needle and a probe pin include the rhenium-tungsten alloy wire according to the embodiment.

## Description

### FIELD

Embodiments described below relate to a rhenium-tungsten alloy wire, a method of manufacturing the same, a medical needle, and a probe pin.

### BACKGROUND

A tungsten alloy (ReW) wire containing a predetermined amount of rhenium (Re) has improved electrical resistance properties and wear resistance as compared with a normal tungsten (W) wire. Tensile strength over a wide range of temperatures and ductility after recrystallization are also improved. Therefore, it is used for probe pins for semiconductor inspection, heaters for electron tubes, filament materials for vibration-resistant bulbs, thermocouples, filaments for fluorescent display tubes, medical needles, and the like.

The ReW wire becomes white silver with metallic luster after the surface mixture layer generated in a manufacturing step is removed by electropolishing or the like, but the surface becomes discolored to, for example, blue, yellow, red purple, or the like as the storage period becomes longer. When discoloration occurs, the electrical resistance of the probe pin may change. In a filament for a display tube, uniformity disappears in coating (electrodeposition surface treatment) of an oxide for emitting thermoelectrons. In a medical needle, when a heat treatment is performed in a discolored state, mechanical properties are deteriorated, and wire breakage may occur during bending processing or the like. In addition, quality deterioration and impurity such as a change in frictional force in a discolored portion and a risk of a discolored portion falling off become problems.

Because of this, the discolored portion cannot be used, and an additional treatment such as re-electrolysis is required. Further, the additional treatment causes a change in size of the wire to change the electrical resistance or strength, and therefore the wire becomes unusable and the yield decreases. Therefore, after the electrolysis step, for example, drying is sufficiently performed, and a wound wire spool is sealed and packed under reduced pressure, and stored in a state of being shielded from the external environment. However, in the ReW wire stored in the same manner, there are variations in the occurrence of discoloration.

### CITATION LIST

### PATENT LITERATURE

Patent Literature 1: Jpn. Pat. Appln. KOKAI Publication No. 4-308003
Patent Literature 2: Jpn. Pat. Appln. KOKAI Publication No. 2021-95585

### NON-PATENT LITERATURE

Non-Patent Literature 1: Saburo Nagakura and five others "Iwanami Dictionary of Physics and Chemistry, 5th Edition", Iwanami Shoten, February 1998, p. 541

### SUMMARY

### TECHNICAL PROBLEM

The problem to be solved by the present invention is to provide a ReW wire that facilitates long-term storage by preventing discoloration.

### SOLUTION TO PROBLEM

In order to solve the above problem, a rhenium-tungsten alloy wire according to an embodiment is a wire made of a tungsten alloy containing rhenium, in which the amount of NH₄ at a wire surface is 10 massppm or less. The amount of NH₄ at the wire surface is determined by a ratio of a weight of NH₄ ions at the wire surface to a weight of an alloy wire (a weight of NH₄ ions at a wire surface/a weight of an alloy wire).

### BRIEF DESCRIPTION OF THE DRAWINGS

FIG. 1a is an example of a result of TDS analysis of a non-discolored portion of a ReW wire.
FIG. 1b is an example of a result of TDS analysis of a discolored portion of a ReW wire.
FIG. 2 is a cross-sectional view of a test apparatus for evaluating an effect of NH₄ on a ReW wire.
FIG. 3 is a graph illustrating discoloration of a ReW wire and a reflection spectrum thereof.
FIG. 4 is an example of a result of analyzing a discolored sample (CW) and a non-discolored sample (NW) by X-ray photoelectron spectroscopy (XPS).
FIG. 5 is an explanatory diagram of a particle size distribution.

### DETAILED DESCRIPTION

Hereinafter, a rhenium-tungsten alloy wire of an embodiment will be described with reference to the drawings. Hereinafter, the rhenium-tungsten alloy wire may be referred to as a ReW wire. Note that the drawings are schematic, and for example, the dimensional proportions of each part are not limited to those shown in the drawings.

The rhenium-tungsten alloy wire according to an embodiment is a wire made of a tungsten alloy containing rhenium, in which the amount of NH₄ at a wire surface is 10 massppm or less. The amount of NH₄ at the wire surface is determined by a ratio of a weight of NH₄ ions at the wire surface to a weight of an alloy wire (a weight of NH₄ ions at a wire surface/a weight of an alloy wire).

Among rhenium oxides, ReaOs is also referred to as dirhenium pentoxide, and is a blue compound. ReO₃ is also referred to as rhenium trioxide, and is a red cubic crystal with metallic luster. Re₂O₇ is also referred to as dirhenium heptoxide, and is a yellow orthorhombic crystal (Non-Patent Literature 1). As described above, the rhenium oxide has a color, and the color changes depending on the type of oxide. As a result of intensive studies, it has been found that the discoloration of a ReW wire is caused by a rhenium oxide and further affected by NH₄.

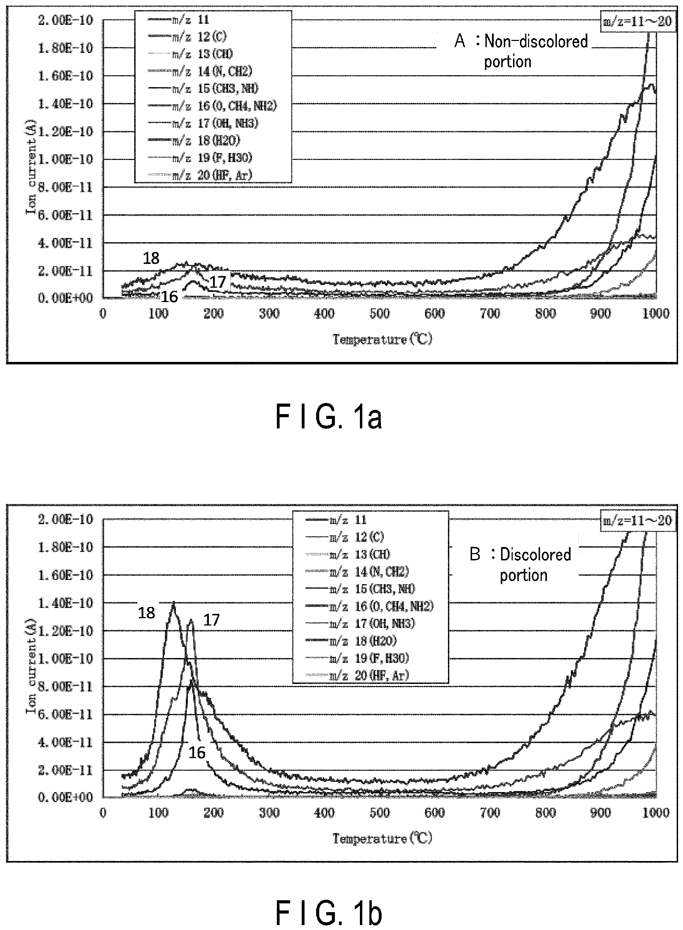

FIGS. 1a and 1b show examples of results of TDS analysis of a non-discolored portion (A) and a discolored portion (B) of a ReW wire. As an apparatus, a TDS1200II quadrupole mass meter manufactured by ESCO Ltd. was used. The length of an evaluation sample was set to 1 cm, and measurement was performed at a measurement temperature in a range of room temperature to 1,400 °C. In B (discolored portion), it was found that desorption of NH₃ (m/z = 17, 16) and H₂O (m/z = 18, 17) was remarkable as compared with a non-discolored portion (A).

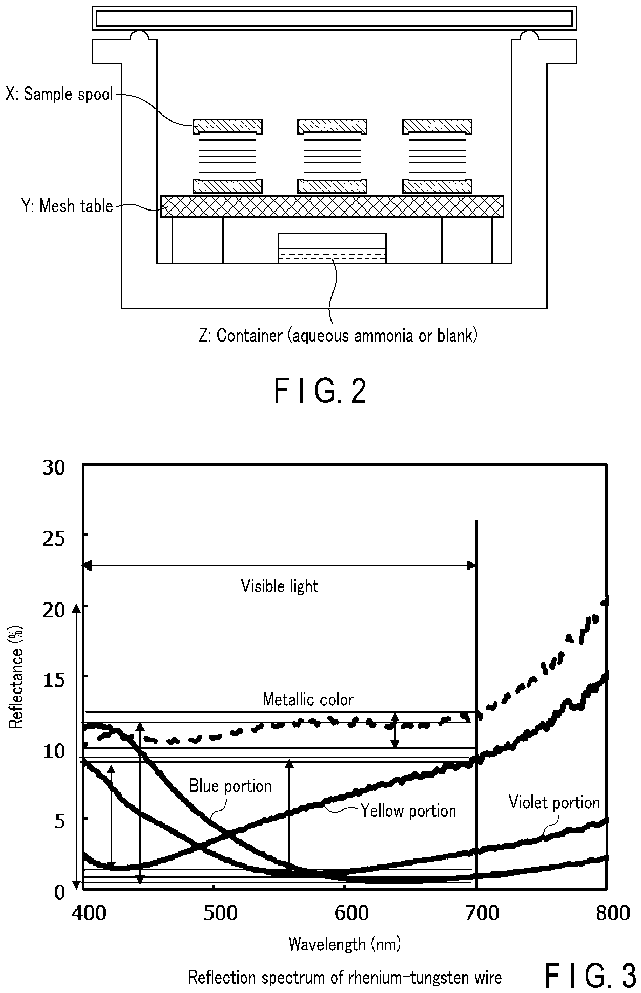

In order to check the effect of NH₄, mandatory testing in which aqueous ammonia is provided in an airtight container was performed. FIG. 2 illustrates a cross section of the test apparatus. A sample (X) which had been subjected to electropolishing and wound around a spool was provided on a mesh table (Y) of the test apparatus, so that the sample (X) was enclosed in the apparatus and left for 4 days. When aqueous ammonia was placed in an airtight container (Z) provided below the mesh table (Y) and left to stand, the ammonia atmosphere concentration in the apparatus was 3.5%.

In Test 1, an electrolyzed and doped tungsten wire without discoloration was used. In Test 2, a wire discolored after storage of a 26% Re-W wire was re-electrolyzed and used. In Test 3, a wire without discoloration after storage of a 26% Re-W wire was re-electrolyzed and used. Table 1 shows the test results. The doped tungsten (W) wire was not discolored regardless of the ammonia atmosphere. In the ReW wire, discoloration occurred only in the ammonia atmosphere, Re affected the discoloration, and the discoloration was promoted by the presence of NH₄. "Without provision of aqueous ammonia" (blank shown in FIG. 2) in Table 1 indicates a case where a test was performed by placing water instead of aqueous ammonia in an airtight container (Z).

**[Table 1]**

| Sample | Type | History before electrolysis | Diameter | Provision of aqueous ammonia | |
|---|---|---|---|---|---|
| | | | (mm) | Without | With |
| Test 1 | Doped W | - | 0.15 | ○ | ○ |
| Test 2 | 26% Re-W | Wire with discoloration | 0.2 | ○ | × |
| Test 3 | 26% Re-W | Wire without discoloration | 0.15 | ○ | × |

| | | | | | |
|---|---|---|---|---|---|
| ∘: without discoloration, × with discoloration | | | | | |

FIG. 3 illustrates discoloration of a ReW wire and a reflection spectrum thereof. The discoloration exhibits the color (blue, purple (red blue), or yellow) of the rhenium oxide described above. The reflection spectrum of each discolored portion was measured under the conditions of a measurement spot of about ϕ 10 µm, an exposure time of 10 ms, and an accumulation count of 200 times using a microspectroscopic system (DF-1037 manufactured by Techno-Synergy, Inc.) configured to incorporate a spectroscopic system into a microscope and enabling spectroscopic measurement of a minute spot. The difference between the maximum value and the minimum value of the reflectance was 5% or less in the case of a white silver color (metallic color) without discoloration with respect to a visible light wavelength of 400 nm to 700 nm. On the other hand, the reflectance of the discolored portion changed to a spectrum having a peak with a difference from the minimum value of 5% or more in a wavelength portion corresponding to each color. As a result, the discolored portion is determined visually or by determining whether the difference between the maximum value and the minimum value of the reflectance in the wavelength of 400 nm to 700 nm of the reflection spectrum exceeds 5%.

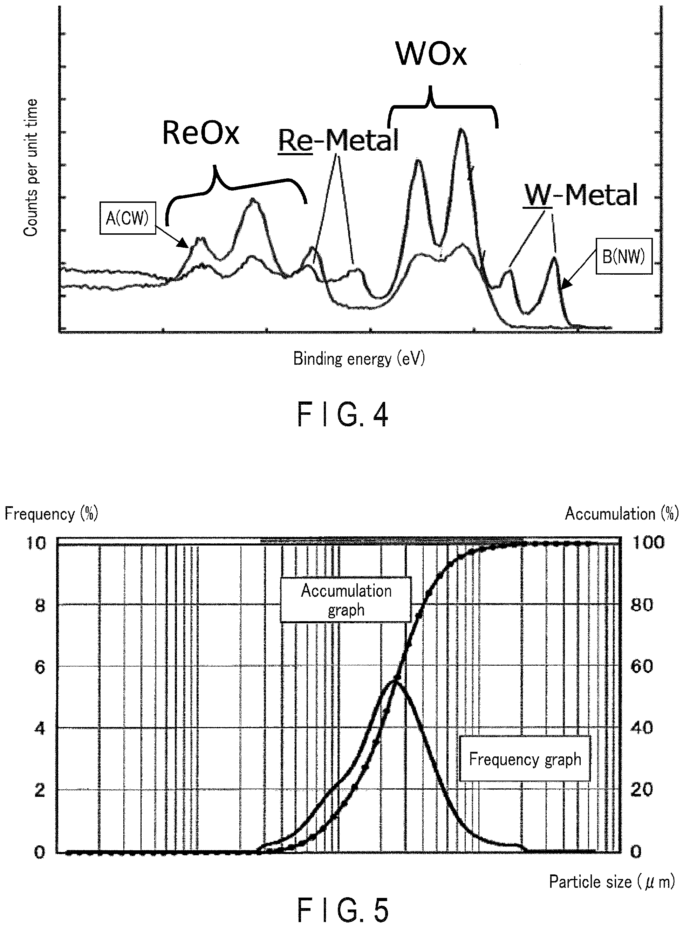

FIG. 4 shows an example of a result of analyzing a discolored sample (CW) and a non-discolored sample (NW) by X-ray photoelectron spectroscopy (XPS) in a 26 wt% Re-W wire of ϕ 0.152 mm stored for a given period after the surface was subjected to electropolishing to have metallic luster. Quantera SXM manufactured by ULVAC-PHI, Inc. is used as an apparatus, an X-ray source is a single crystal spectroscopic A1Kα ray, an X-ray output is 12.5 W, and an analysis range is ϕ 50 um.

In NW (non-discolored sample) denoted by B in FIG. 4, more W-Metal and tungsten oxide (WOₓ) were detected than in CW (discolored sample) denoted by A in FIG. 4, and in CW denoted by A in FIG. 4, more rhenium oxide (ReOₓ) was detected than in NW denoted by B in FIG. 4. As described above, the rhenium oxide has a color, and the color changes depending on the type of oxide. That is, it was found that the rhenium oxide is a cause of discoloration, and the presence of NH₄ promotes rhenium oxide formation.

Table 2 shows the results of measuring the amount of NH₄ in the discolored and non-discolored portions by hot water extraction-ion chromatography. The amount of NH₄ was measured by ion chromatography after hot water extraction from 0.5 g of the alloy wire into 50 ml of pure water. As an apparatus, ICS-2000 manufactured by Thermo Fischer Scientific Inc. was used. As a result, it was found that discoloration can be prevented by limiting the NH₄ adhesion amount at the surface. Sample 1 in Table 2 corresponds to the discolored sample (A(CW)) with results shown in FIG. 4, and Samples 2, 3, and 4 correspond to the non-discolored sample (B(NW)) with results shown in FIG. 4.

The reason why ammonium ions are mixed into the rhenium-tungsten alloy wire is presumed to be as follows. Ammonia contained in the atmosphere during the step of manufacturing the rhenium-tungsten alloy wire is adsorbed. Ammonia in the atmosphere is generated when organic substances such as animals and plants and ammonium salts are decomposed by bacteria or the like, and this is presumed to be mainly due to spontaneous generation in soil, the ocean, and the like. In addition, in urban areas, generation in sewer systems, human waste treatment plants, chemical plants, and oil refineries, and generation due to combustion of fossil fuels are also considered. In addition, as a lubricant used in drawing processing of the manufacturing method described later, a turbid liquid obtained by dispersing graphite powder in water can be used. Aqueous ammonia is added to the lubricant for pH adjustment. These are presumed to be the cause.

**[Table 2]**

| Sample No. | 1 | 2 | 3 | 4 |
|---|---|---|---|---|
| Discoloration | With | Without | Without | Without |
| NH₄ (massppm) | 11 | <5 | <5 | <5 |

In the rhenium-tungsten alloy wire of the embodiment, the amount of NH₄ at a wire surface is 10 massppm or less, preferably 5 massppm or less. The amount of NH₄ at the wire surface is determined by a ratio of the weight of NH₄ ions at the wire surface to the weight of an alloy wire (the weight of NH₄ ions at a wire surface / the weight of an alloy wire). Reduction of the amount of NH₄ present at the surface to 10 massppm or less, and further to 5 massppm or less enables prevention of discoloration, and enables manufacturing with a high yield regarding the use as a material for a probe pin or a medical needle.

The amount of NH₄ at the wire surface can be set to a target value by, for example, adjusting the surface treatment of the rhenium-tungsten alloy wire, for example, electropolishing conditions, washing conditions, or drying conditions of the rhenium-tungsten alloy wire. In addition, even if a method of storing the rhenium-tungsten alloy wire or a method of packing the rhenium-tungsten alloy wire is adjusted, the amount of NH₄ at the wire surface can be set to a target value. The adjustment may be performed by each of the above methods alone or by combining a plurality of methods.

The content of rhenium contained in the ReW wire of the embodiment is, for example, 2 wt% or more and less than 30 wt%. In addition, the content of rhenium contained in the ReW wire of the embodiment is preferably, for example, 10 wt% or more and 28 wt% or less. The rhenium content is a value analyzed by inductively coupled plasma-optical emission spectrometry (ICP-OES). Rhenium improves the elongation of tungsten at high temperatures and enhances the workability. The strength is also increased by solid solution strengthening.

When the rhenium content is less than 2 wt%, the effect is insufficient. For example, in a case where a ReW wire having a rhenium content of less than 2 wt% is used as a material for a probe pin, the amount of deformation of the probe pin increases with the frequency of use and failure in contact occurs to degrade the inspection accuracy of a semiconductor. When the rhenium content is 30 wt% or more, the limit of solid solubility with tungsten is exceeded, and thus it becomes impossible to diffuse and homogenize (to form a solid solution of) rhenium in the tungsten matrix. As a result, a phase region in which the composition ratio of rhenium is locally high (a segregated phase of a σ phase) may occur. When such a portion appears at the surface, discoloration is likely to occur.

When a probe pin or a medical needle is manufactured using a ReW wire having a rhenium content of 2 wt% or more and less than 30 wt% as a material, discoloration can be prevented and manufacturing can be performed with a high yield, and the mechanical properties (strength and wear resistance) of the manufactured probe pin or medical needle can be ensured. The rhenium content is preferably, for example, 10 wt% or more and 28 wt% or less.

The ReW wire of the embodiment may contain potassium (K) as a dopant in an amount of 30 wtppm or more and 90 wtppm or less. The potassium content is a value analyzed by inductively coupled plasma-optical emission spectrometry (ICP-OES). Incorporation of potassium improves tensile strength and creep strength at high temperatures due to the doping effect.

When the potassium content is less than 30 wtppm, the doping effect becomes insufficient. When the potassium content exceeds 90 wtppm, the workability may decrease to cause a large decrease in the yield. When potassium is contained as a dopant in an amount of 30 wtppm or more and 90 wtppm or less, for example, thin wires for a thermocouple or for an electron tube heater made of the ReW wire of the embodiment can be manufactured with a high yield while ensuring high-temperature properties (prevention of wire breakage and deformation during high-temperature use).

The ReW wire of the embodiment has, for example, a diameter of 0.1 mm or more and 1.00 mm or less.

The ReW wire of such an embodiment prevents surface discoloration and greatly contributes to long-term storage and improvement in yield. In addition, the ReW wire of the embodiment can be applied to a medical needle. The present invention can also be applied to a ReW wire for a thermocouple or a probe pin.

Next, a method of manufacturing the ReW wire according to the embodiment will be described. The manufacturing method is not particularly limited, and examples thereof include the following methods.

A tungsten powder and a rhenium powder are mixed so that the rhenium content is 2 wt% or more and less than 30 wt%. The mixing method is not particularly limited, but a method of mixing the powders with water or an alcohol-based solution to form a slurry is particularly preferable because powders with good dispersibility can be obtained. In addition, in order to ensure the homogeneity of the powder lots, it is more preferable to dry the above-described slurry and then collectively stir the same powder lots in a dry state.

The rhenium powder to be mixed preferably has an average particle size of less than 8 um. The particle size distribution preferably has an SD value of less than 11 µm. FIG. 5 is an explanatory diagram of the particle size distribution. The horizontal axis represents particle size (µm), the left vertical axis represents frequency (%), and the right vertical axis represents accumulation (%). The SD value is a value determined by SD = (d (84%) - d(16%))/2 where d(84%) denotes a particle size at an accumulation of 84% and d(16%) denotes a particle size at an accumulation of 16%. The SD is a guide for a distribution width of the measured particle size. The particle size distribution is measured by a laser diffraction method. The amount of powder used for single measurement is the amount recommended by the measurement apparatus. In general, 0.02 g is recommended. In addition, the measurement sample shall be sufficiently stirred before measurement and then weighed.

The tungsten powder is a pure tungsten powder excluding inevitable impurities or a doped tungsten powder containing potassium (K) in an amount in consideration of the yield up to the wire material. The tungsten powder preferably has an average particle size of less than 16 um. The particle size distribution preferably has an SD value of less than 13 um. When the average particle size of the rhenium powder is 8 µm or more, when the SD value of the rhenium powder is 11 µm or more, when the average particle size of the tungsten powder is 16 µm or more, or when the SD value of the tungsten powder is 13 µm or more, a diffusion distance of rhenium atoms or tungsten atoms for diffusing and homogenizing (forming a solid solution of) rhenium in the tungsten matrix increases, and a σ phase is easily formed.

The ratio of the average particle size of the rhenium powder to the average particle size of the tungsten powder (Re average particle size/W average particle size) is preferably 0.4 or more and 2.0 or less. When the ratio of the average particle size of the rhenium powder to the average particle size of the tungsten powder is less than 0.4 or more than 2.0, the diffusion distance of the rhenium atoms to the center of the tungsten particles or the diffusion distance of the tungsten atoms to the center of the rhenium particles increases, and a σ phase may be easily generated.

Subsequently, the mixed powder is placed in a predetermined mold and press-molded. The pressing pressure at this time is preferably 150 MPa or more. The molded body may be subjected to a presintering treatment at 1,200 to 1,400 °C in a hydrogen furnace in order to facilitate handling. The obtained molded body is sintered in a hydrogen atmosphere, an inert gas atmosphere such as argon, or under vacuum. The sintering temperature is preferably 2,500 °C or higher. When the sintering temperature is lower than 2,500 °C, diffusion of rhenium atoms and tungsten atoms does not sufficiently proceed during sintering. The upper limit of the sintering temperature is 3,400 °C (the melting point of tungsten is 3,422 °C or lower).

The relative density of the sintered body (relative density (%) to the true density = [sintered body density/true density] × 100%) is preferably 90% or more. In addition, in one sintered body, for example, the ratio of the density of the lowest portion such as the lower end in electric sintering to the average density of the entire sintered body is preferably 0.98 or more. The variation in the rhenium content can be reduced by setting the relative density of the sintered body to 90% or more and setting the ratio of the density of the lowest portion to the average density of the entire sintered body to 0.98 or more.

The sintered body obtained in the main sintering step is subjected to first swaging (SW) processing. The first swaging processing is preferably performed at a heating temperature of 1,300 to 1,600 °C. The reduction rate of the cross-sectional area (area reduction rate) at which processing is performed by a single heat treatment (single heating) is preferably 5 to 15%.

Instead of the first swaging processing, rolling processing may be performed. The rolling processing is preferably performed at a heating temperature of 1,200 to 1,600 °C. The area reduction rate in single heating is preferably 40 to 75%. As a rolling mill, a two-way roller rolling mill, a four-way roller rolling mill, a die roll rolling mill, or the like can be used. The rolling processing can greatly enhance the manufacturing efficiency. The first swaging (SW) processing and the rolling processing may be combined.

The sintered body (ReW rod) after completion of the first swaging processing, the rolling process, or the combined processing of the first swaging processing and the rolling processing is subjected to second swaging (SW) processing. The second swaging processing is preferably performed at a heating temperature of 1,200 to 1,500 °C. The area reduction rate in single heat (single heating) is preferably about 5 to 20%.

Subsequently, the ReW rod after completion of the second swaging step is subjected to a recrystallization treatment. The recrystallization treatment can be performed at a treatment temperature in a range of 1,800 to 2,600 °C in a hydrogen atmosphere, in an inert gas atmosphere such as argon, or under vacuum using, for example, a high-frequency heating apparatus.

The ReW rod after completion of the recrystallization treatment is subjected to third swaging processing. The third swaging processing is preferably performed at a heating temperature of 1,200 to 1,500°C. The area reduction rate in single heating is preferably about 10 to 30%. The third swaging processing is performed until the ReW rod has a diameter allowing drawing processing (preferably, a diameter of 2 to 4 mm) .

In order to enable smooth drawing (DW) processing, the ReW rod after completion of the third swaging processing is subjected to a treatment of applying a lubricant to the surface, and the lubricant is dried. In the drawing processing, application of a lubricant, drying of the lubricant, a treatment of heating to a temperature that allows processing, and a treatment of drawing using a drawing die are repeated. It is desirable to use a carbon (C)-based lubricant excellent in heat resistance as the lubricant. The processing temperature is set according to the wire diameter to be obtained by the drawing processing. The processing temperature is preferably, for example, 1,100 °C or lower. The area reduction rate per die is preferably 10 to 35%. If necessary, an annealing step or a surface polishing step (for example, an electrolysis step) may be added under known conditions during the drawing step.

The ReW wire after completion of the drawing step is subjected to polishing processing. As the polishing processing, for example, there is a method of electrochemically polishing (electropolishing) in an aqueous sodium hydroxide solution at a concentration of 3 to 15 wt%. The area reduction rate in the polishing processing is preferably 10 to 25%. When the area reduction rate is less than 10%, protrusions and recesses on the material surface generated in the swaging step or the drawing step and the mixture adhering to the protrusions and recesses on the material surface may not be able to be removed. When the surface removal amount is insufficient, the mixture layer locally remains, and thus the abundance ratio of rhenium may increase. When the area reduction rate exceeds 25%, the material yield deteriorates.

In the case of electropolishing, the polishing speed is preferably 0.5 to 3.0 µm/sec. When the polishing speed is slower than 0.5 µm/sec, tungsten at the surface may be preferentially dissolved to increase the abundance ratio of rhenium at the surface. When the polishing speed exceeds 3.0 µm/sec, the amount of electrolysis per unit time increases, leading to rapid electrolysis, and correction of the cross-sectional shape of the ReW wire may become insufficient. The ReW wire after electropolishing is washed to remove an alkali metal, carbon, or the like remaining on the surface. The washing water is preferably pure water having a conductivity of 1 µS/cm or less. Ultrasonic washing may be used in combination.

The ReW wire after completion of the polishing processing may be subjected to, for example, a drying step. The drying step is performed by, for example, a vacuum dryer whose internal temperature is set within a range of 50 to 80 °C. The drying time is, for example, 1 hour or more. Thereafter, a predetermined shipping inspection is performed. When the ReW wire after vacuum drying is stored, for example, moisture adsorption can be prevented by housing the ReW wire in a moisture-proof storage having a relative humidity of 5% or less. For example, the ReW wire is housed and stored in the moisture-proof storage except when property or quantity inspection or the like is performed.

The ReW wire is wound around a spool for shipping while, for example, shipping inspection is performed. In the ReW wire after completion of the shipping inspection, for example, the outermost surface wound around the spool for shipping is covered with a protective paper, and the protective paper was fixed with a rubber band or the like. Thereafter, for example, the ReW wire is housed in an aluminum bag together with an ammonia adsorbent (for example, containing SiO₂ as a main component) in a necessary amount according to the volume of the aluminum bag, and the aluminum bag is subjected to a degassing treatment to such an extent that no tension is applied to the aluminum bag, and then sealed.

A probe pin or a medical needle having a predetermined wire diameter and necessary properties (such as strength and hardness) is obtained by performing a necessary step under a known condition using an appropriate amount of the ReW wire obtained in the above-described steps.

### (Example 1)

A doped tungsten powder containing potassium (K) in an amount of 50 wtppm to 80 wtppm in the final wire material and having an average particle size of 13 µm and an SD value of 12 µm and a rhenium powder having an average particle size of 6 um and an SD value of 7 µm were formed into a slurry using an alcohol-based solution and mixed so that the proportion of rhenium is 3 wt%. The obtained slurry was dried to form a raw material powder.

The mixed powder of the raw materials was press-molded to obtain a molded body. The molded body was subjected to a presintering treatment at 1,300 °C in a hydrogen furnace. The molded body was sintered at 3,000 °C in a hydrogen atmosphere to obtain a sintered body. The sintered body was subjected to first swaging processing at a heating temperature of 1,400 °C and a reduction rate of a cross-sectional area processed by a single heat treatment of 14%. The sintered body which was subjected to the first swaging processing was subjected to second swaging processing at a heating temperature of 1,300 °C and a reduction rate of a cross-sectional area processed by a single heat treatment of 15%.

The ReW rod after completion of the second swaging processing was subjected to a recrystallization treatment at a treatment temperature of 2,400 °C in a hydrogen atmosphere. After the recrystallization treatment, third swaging processing was performed at a heating temperature of 1,300 °C and a reduction rate of the cross-sectional area processed by a single heat treatment of 13% to obtain a rod having a diameter of 2.5 mm.

A lubricant was applied to the surface of the rod which was subjected to the third swaging processing, and dried. The obtained rod was subjected to drawing processing. The drawing processing was performed at 1,000 °C so that the reduction rate of the cross-sectional area in single drawing processing was 10% to 35%. In the drawing processing, an annealing step was performed at 1,300 °C.

The ReW wire after completion of the drawing processing was subjected to electropolishing in an aqueous sodium hydroxide solution at a concentration of 8 wt%. The electropolishing step was performed at a reduction rate of the cross-sectional area in the electropolishing step of 15 to 20% and a polishing speed of 2.2 um/sec. After the electropolishing, the ReW wire was washed with pure water having a conductivity of 1 µS/cm. The obtained ReW wire had a wire diameter of 0.8 mm.

ReW after the electropolishing was subjected to a drying step for 2 hours by a vacuum dryer having an internal temperature of 70 °C, and then housed in a moisture-proof storage having a relative humidity of 5% or less. The obtained ReW wire was wound around a spool for shipping of 100 m/spool, and the outermost surface was covered with a protective paper, and the protective paper was fixed with a rubber band. The spool for shipping around which the ReW wire was wound was housed in an aluminum bag together with an ammonia adsorbent containing SiO₂ as a main component, and the aluminum bag was subjected to a degassing treatment to such an extent that no tension was applied to the aluminum bag, and then sealed.

Three spools similar thereto were manufactured, and the samples of the three spools were stored for 13 months by placing the samples side by side on a table having a height of 1 m provided in a room at a humidity of 60% or less and a room temperature of 30 °C or lower. After a lapse of 13 months, whether discoloration occurred was checked by the above-described method (the difference between the maximum value and the minimum value of the reflectance in the wavelength of 400 nm to 700 nm of the reflection spectrum described with reference to FIG. 3).

### (Example 2)

A ReW wire was manufactured in the same manner as in Example 1 except that the tungsten powder as a raw material was not doped with potassium, the ratio of rhenium was 26 wt%, the wire diameter of the final ReW wire was 0.15 mm, and the ReW wire was wound around a spool for shipping of 500 m/spool and housed in an aluminum bag together with an ammonia adsorbent by a packing method similar to that in Example 1. As in Example 1, three identical spools were manufactured, and stored for 13 months, and after a lapse of 13 months, whether discoloration occurred was checked in the same manner as in Example 1.

### (Example 3)

A ReW wire was manufactured by performing steps similar to those in Example 1 up to the drawing processing step using raw material powders similar to those in Example 1. An ReW wire having a wire diameter of 0.8 mm was obtained in the same manner as in Example 1 except that the obtained ReW wire was subjected to the electropolishing step at a polishing speed of 4.0 µm/sec and the ReW wire was not stored in a moisture-proof storage after the electropolishing step. The obtained ReW wire was wound around a spool for shipping of 100 m/spool, and the outermost surface was covered with a protective paper, and the protective paper was fixed with a rubber band. The spool for shipping around which the ReW wire was wound was housed in an aluminum bag together with a desiccant containing silica gel, and the inside of the aluminum bag was subjected to a vacuum degassing treatment and sealed. Three identical spools were manufactured, and stored for 13 months, and after a lapse of 13 months, whether discoloration occurred was checked in the same manner as in Example 1.

### (Comparative Example 2)

A ReW wire was manufactured by performing steps similar to those in Example 2 up to the drawing processing step using raw material powders similar to those in Example 2. A ReW wire having a wire diameter of 0.15 mm was obtained in the same manner as in Example 2 except that the obtained ReW wire was subjected to the electropolishing step at a polishing speed of 4.0 µm/sec and the ReW wire was not stored in a moisture-proof storage after the electropolishing step. The obtained ReW wire was wound around a spool for shipping of 500 m/spool and packed in the same manner as in Example 3. Three identical spools were manufactured and stored for 13 months, and after a lapse of 13 months, whether discoloration occurred was checked in the same manner as in Example 1.

### (Example 4)

A ReW wire was manufactured in the same manner as in Example 2 and wound around a spool for shipping of 500 m/spool. The spool for shipping of Example 4 was housed in an aluminum bag together with a desiccant containing silica gel, and the inside of the aluminum bag was subjected to a vacuum degassing treatment and sealed. Three identical spools were manufactured and stored for 13 months, and after a lapse of 13 months, whether discoloration occurred was checked in the same manner as in Example 1.

Table 3 shows the measurement results of each of the rhenium content, the potassium content, and the amount of NH₄. The analysis of the rhenium content and the potassium content was performed by inductively coupled plasma-optical emission spectrometry (ICP-OES). The lower detection limit of potassium is 5 wtppm, and a case where the analytical value is less than 5 wtppm without addition is indicated by "-". The amount of NH₄ was measured by ion chromatography after hot water extraction from 0.5 g of the alloy wire into 50 ml of pure water. As an apparatus, ICS-2000 manufactured by Thermo Fischer Scientific Inc. was used.

**[Table 3]**

| Sample | Wire diameter | Re | K | Without discoloration: ○ | NH₄ |
|---|---|---|---|---|---|
| | (mm) | (wt%) | (wtppm) | With discoloration: × | (massppm) |
| Example 1 | 0.8 | 3 | 68 | ○ | <5 |
| | | | | ○ | <5 |
| | | | | ○ | <5 |
| Example 2 | 0.15 | 26 | - | ○ | <5 |
| | | | | ○ | <5 |
| | | | | ○ | <5 |
| Example 3 | 0.8 | 3 | 62 | ○ | 9 |
| | | | | × | 20 |
| | | | | × | 15 |
| Comparative Example 2 | 0.15 | 26 | - | × | 14 |
| | | | | x | 18 |
| | | | | × | 17 |
| Example 4 | 0.15 | 26 | - | × | 11 |
| | | | | × | 12 |
| | | | | ○ | 5 |

As can be seen from Table 3, the ReW wire according to the embodiment can prevent discoloration even in long-term storage, and can greatly improve the yield in use as a probe pin or a medical needle.

In Examples 1 to 4 in which the amount of NH₄ at the wire surface is 10 massppm or less in at least one of the 3 spools, discoloration after long-term storage could be prevented. On the other hand, in Comparative Example 2 in which the amount of NH₄ at the wire surface exceeds 10 massppm in all of the 3 spools, discoloration occurred due to long-term storage. Although it is inevitable that a slight variation will occur in the amount of moisture or the adsorption amount of ammonia in the working environment after the electropolishing step until the wire is housed in the aluminum bag, it is presumed that Example 3 and Comparative Example 2 are easily affected by the variation in the amount of moisture or the adsorption amount of ammonia. This is considered to be because, in Example 3 and Comparative Example 2, the electropolishing speed was set to 4.0 µm/sec, the wire was not stored in a moisture-proof storage, and also no ammonia adsorbent was used.

While some embodiments of the present invention have been illustrated above, these embodiments have been presented as examples, and are not intended to limit the scope of the invention. These novel embodiments can be implemented in various other forms, and various omissions, substitutions, changes, and the like can be made without departing from the gist of the invention. These embodiments and their modifications are included in the scope and gist of the invention, and are included in the invention described in the claims and their equivalents. In addition, the above-described embodiments can be implemented in combination with each other.

Hereinafter, the inventions of the embodiments will be additionally described.
<1>. A rhenium-tungsten alloy wire which is a wire comprising a tungsten alloy containing rhenium, wherein an amount of NH₄ at a wire surface is 10 massppm or less, and is according to a ratio of a weight of NH₄ ions at the wire surface to a weight of an alloy wire.
<2>. The rhenium-tungsten alloy wire according to <1>, wherein a rhenium content is 2 wt% or more and less than 30 wt%.
<3>. The rhenium-tungsten alloy wire according to <1>, wherein the rhenium content is 10 wt% or more and 28 wt% or less.
<4>. The rhenium-tungsten alloy wire according to any one of <1> to <3>, wherein a potassium (K) content is 30 wtppm or more and 90 wtppm or less.
<5>. The rhenium-tungsten alloy wire according to any one of <1> to <4>, which has a diameter of 0.1 mm or more and 1.00 mm or less.
<6>. The rhenium-tungsten alloy wire according to any one of <1> to <5>, which is used for a wire material of a medical needle.
<7>. The rhenium-tungsten alloy wire according to any one of <1> to <5>, which is used for a wire material of a probe pin.
<8>. A method of manufacturing the rhenium-tungsten alloy wire according to any one of <1> to <7>.
<9>. A medical needle comprising the rhenium-tungsten alloy wire according to any one of <1> to <5>.
10. A probe pin comprising the rhenium-tungsten alloy wire according to any one of <1> to <5>.

### REFERENCE SIGNS LIST

- A: Non-discolored portion
- B: Discolored portion
- X: Sample spool
- Y: Mesh table
- Z: Container

- A(CW): Discolored sample
- B(NW): Non-discolored sample

## Claims

1. A rhenium-tungsten alloy wire which is a wire comprising a tungsten alloy containing rhenium, wherein an amount of NH₄ at a wire surface is 10 massppm or less, and is according to a ratio of a weight of NH₄ ions at the wire surface to a weight of an alloy wire.

2. The rhenium-tungsten alloy wire according to claim 1, wherein a rhenium content is 2 wt% or more and less than 30 wt%.

3. The rhenium-tungsten alloy wire according to claim 1, wherein the rhenium content is 10 wt% or more and 28 wt% or less.

4. The rhenium-tungsten alloy wire according to claim 1, wherein a potassium (K) content is 30 wtppm or more and 90 wtppm or less.

5. The rhenium-tungsten alloy wire according to claim 1, which has a diameter of 0.1 mm or more and 1.00 mm or less.

6. The rhenium-tungsten alloy wire according to any one of claims 1 to 5, which is used for a wire material of a medical needle.

7. The rhenium-tungsten alloy wire according to any one of claims 1 to 5, which is used for a wire material of a probe pin.

8. A method of manufacturing the rhenium-tungsten alloy wire according to any one of claims 1 to 5.

9. A medical needle comprising the rhenium-tungsten alloy wire according to any one of claims 1 to 5.

10. A probe pin comprising the rhenium-tungsten alloy wire according to any one of claims 1 to 5.
